Europäisches Patentamt

European Patent Office

Office européen des brevets

(19)

(11) Veröffentlichungsnummer: **0 083 028**
**B1**

(12) # EUROPÄISCHE PATENTSCHRIFT

(45) Veröffentlichungstag der Patentschrift:
05.08.87

(51) Int. Cl.⁴: **A 61 C 8/00, A 61 F 2/30**

(21) Anmeldenummer: **82111636.5**

(22) Anmeldetag: **15.12.82**

(54) Verbindungselement für einen Implantatkörper und eine Suprastruktur.

(30) Priorität: **16.12.81 DE 3149881**

(43) Veröffentlichungstag der Anmeldung:
**06.07.83 Patentblatt 83/27**

(45) Bekanntmachung des Hinweises auf die Patenterteilung:
**05.08.87 Patentblatt 87/32**

(84) Benannte Vertragsstaaten:
**AT BE CH DE FR GB IT LI NL SE**

(56) Entgegenhaltungen:
**EP - A - 0 015 599
CH - A - 237 877
DE - A - 2 139 683
DE - A - 2 249 051
DE - A - 2 413 883
DE - A - 2 704 390
DE - A - 2 824 214
DE - A - 2 830 025
DE - A - 2 905 647
DE - A - 2 950 219
US - A - 3 589 011
US - A - 3 934 347**

**Die Akte enthält technische Angaben, die nach dem Eingang der Anmeldung eingereicht wurden und die nicht in dieser Patentschrift enthalten sind.**

(73) Patentinhaber: **FRAUNHOFER-GESELLSCHAFT ZUR FÖRDERUNG DER ANGEWANDTEN FORSCHUNG E.V., Leonrodstrasse 54, D-8000 München 19 (DE)**
Patentinhaber: **Scheicher, Hans, Dr.med. Dr.med.dent., Rondell Neuwittelsbach 4, D-8000 München 19 (DE)**

(72) Erfinder: **Scheicher, Hans Dr. med. Dr. med. dent., Rondell Neuwittelsbach 4, D-8000 München 19 (DE)**
Erfinder: **Siegele, Dieter, Dipl.-Ing., Häge 6, D-7800 Freiburg (DE)**
Erfinder: **Soltész, Uwe, Dr.-Ing. Dipl.-Phys., Im Laimacker 48, D-7802 Merzhausen (DE)**

(74) Vertreter: **Kraus, Walter, Dr. et al, Patentanwälte Kraus, Weisert & Partner Thomas-Wimmer-Ring 15, D-8000 München 22 (DE)**

**Beschreibung**

Die Erfindung betrifft ein Verbindungselement für einen mit einer Bohrung versehenen Implantatkörper mit einer Suprastruktur, wobei das Verbindungselement einen Stift hoher Biegefestigkeit aufweist, dessen Durchmesser kleiner als derjenige der Bohrung ist und der in dem Hohlraum zwischen dem Stift und der Wand des Implantatkörpers mit letzterem in Verbindung steht.

Bei vielen enossalen Implantaten (im Knochen verankerter alloplastischer Zahnwurzelersatz) kommt es häufig nach kurzer bis mehrjähriger Tragezeit zu einem Knochenabbau im Implantatlager und dadurch zu einer Auslockerung, die schliesslich zum Verlust des Implantats oder sogar zu seinem Bruch führt.

Diese sogenannte «Resorption» des Knochens kann nur zum Teil auf mangelnde Biokompatibilität (Gewebsverträglichkeit) des Implantatmaterials oder auf Infektionen zurückgeführt werden. Eine wesentliche Ursache für den Knochenabbau ist die biomechanische Überlastung des Implantatlagers. Eine lokale, sich häufig wiederholende Belastung des Knochens mit mechanischen Spannungsspitzen führt zur Rückbildung des Knochens. Dies trifft sowohl für dentale Wurzelimplantate wie auch für Knochen- und Gelenkimplantate zu. Bei der Einpflanzung derartiger Implantate ist es daher für deren dauerhaften Bestand im Organismus von ausschlaggebender Bedeutung, dass durch geeignete Kraftumverteilungsmechanismen diese schädlichen Spannungsspitzen vermieden werden.

Bei Implantaten für den Zahnwurzelersatz, wie auch bei anderen Implantatarten, gibt es eine Reihe von Belastungssituationen, die bei einem direkten Kraftschluss zwischen der angreifenden Last und dem Implantatlager über ein starres, fest sitzendes Implantat zu derartigen lokalen Spannungsspitzen und somit zur Resorption, Auslockerung und zum Verlust des Implantats führen.

Der natürliche Zahn ist durch ein elastisches Fasergeflecht, das Periodontium, hängemattenartig aufgehängt und mit dem umgebenden Knochen verbunden. Durch diesen Mechanismus kann der Zahn sowohl bei Einwirkung von horizontalen als auch von vertikalen Kräften elastisch ausgelenkt werden, wobei das Knochenlager über die Fasern wahrscheinlich weitgehend gleichmässig und überwiegend auf Zug beansprucht wird. So beträgt die physiologische Auslenkung eines oberen Schneidezahns zum Beispiel bei Einwirkung einer horizontalen Last von 5 N ca. 20 bis 80 μm. Bei Einwirkung einer gleich grossen vertikalen Kraft wird die Zahnwurzel ca. 20 bis 30 μm in das Zahnfach eingedrückt. Gleichzeitig erfolgt durch Mechanorezeptoren im Periodontium über corticale und subcorticale Steuerungsmechanismen (Cerebralnerven) eine Koordinierung und Begrenzung der Kraftfreisetzung der Kaumuskulatur.

Bei einem enossalen Implantat liegt dieses dentale Sensorium nicht vor und somit auch keine Steuerung der Krafteinwirkung, so dass es allein schon hierdurch zu Überlastungen kommen kann.

Ausserdem ist ein Implantat zumeist formschlüssig vom Knochen umwachsen oder eventuell sogar kraftschlüssig mit dem Knochen verwachsen. Bei einem starren Verbund zwischen dem Implantat und dem Zahn- oder Brückenersatz wird die gesamte angreifende Kraft direkt und örtlich begrenzt über das Implantat auf das Implantatlager übertragen. Beim natürlichen Zahn dagegen kommt es beim punktförmigen Angreifen einer Last zunächst zur Auslenkung bzw. zum Einsinken des Zahnes und durch eine sinnvolle Beziehung der Zahnreihen und der Zahnhöcker zueinander sehr schnell zu einer Abstützung der Kaukräfte durch die übrigen Zähne. Eine Überlastung des einzelnen Zahnhalteapparats wird dadurch vermieden. Beim form- und kraftschlüssig eingewachsenen Implantat besteht diese Auslenkmöglichkeit nicht, es kommt zu Spannungsüberhöhungen im Knochenlager und damit zum Knochenabbau und zum Verlust des Implantats.

Durch spannungsoptische Untersuchungen und numerische Berechnungen konnte ermittelt werden, dass in erster Linie die Horizontalkomponenten der am Zahn angreifenden Kräfte zu erheblichen Spannungsspitzen, insbesondere am Austrittsrand des Implantats aus dem Knochen, in der sogenannten «Corticalis» führen. Dies erklärt die immer wieder zu beobachtende trichterförmige Öffnung und Rückbildung des Knochens um das Implantat.

Bei vertikaler Belastung werden die Kräfte weitgehend symmetrisch über die Wandung des Implantats und über den Implantatboden in den Knochen eingeleitet. Zu lokalen Spannungsüberhöhungen kommt es dabei normalerweise nicht, eine generelle Überlastung des gesamten Implantatbettes kann jedoch ebenfalls auftreten, da das Implantat im Gegensatz zum natürlichen Zahn starr mit dem Knochen verbunden ist.

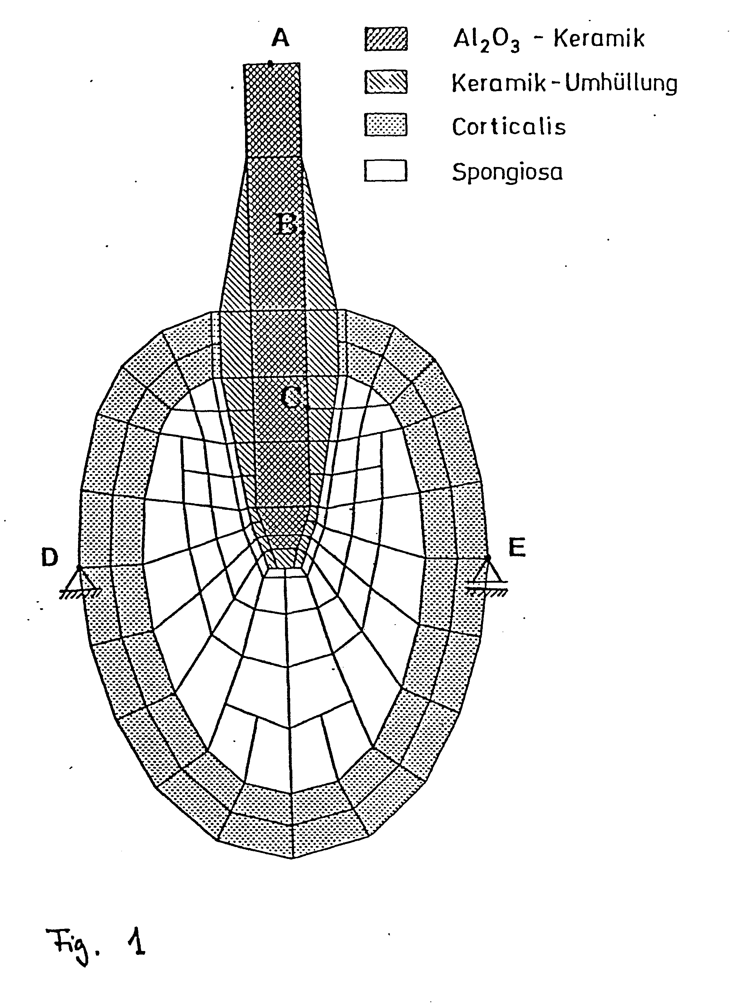

In den Figuren 1 bis 4 sind die Ergebnisse von Modellrechnungen (Finite-Element-Berechnungen), die am Fraunhofer-Institut für Werkstoffmechanik in Freiburg durchgeführt wurden, dargestellt.

Figur 1 zeigt schematisch das FE-Modell eines Implantats im Unterkiefer unter Annahme eines festen Verbunds zwischen Implantat und Knochenlager.

Dargestellt ist ein Querschnitt durch den Kiefer und das Implantat. Der Knochen ist inhomogen und besteht aus der harten Corticalisschale und der weichen inneren Spongiosa. Der Elastizitätsmodul (E-Modul) der Corticalis ist mit 20 000 N/mm$^2$, der der Spongiosa mit 2000 N/mm$^2$ und der des Implantats (Al$_2$O$_3$-Keramik) mit 380 000 N/mm$^2$ angenommen.

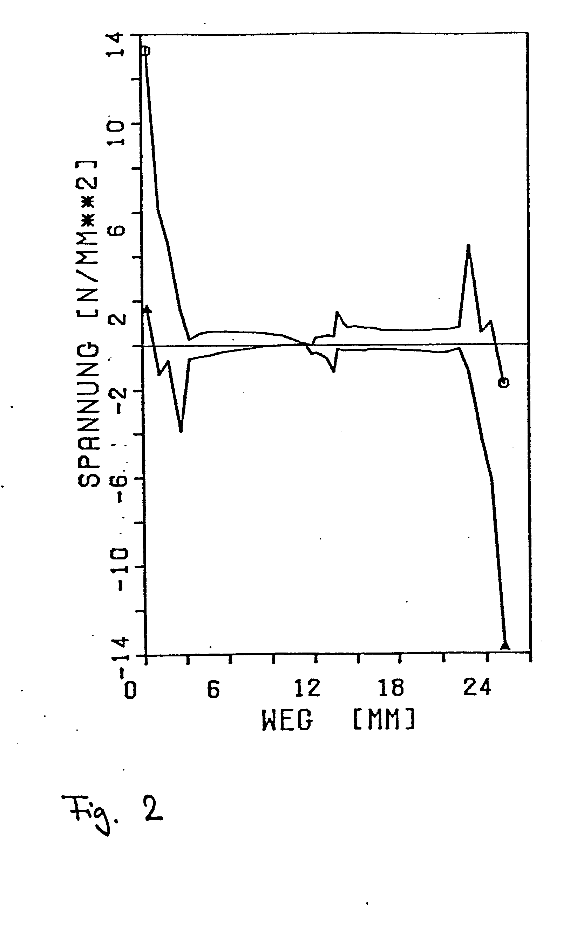

Figur 2 zeigt die Verteilung der Hauptspannungen im Knochen entlang der Grenzfläche zum Implantat, wobei die Abszisse den Weg entlang der Implantatoberfläche entgegen dem Uhrzeigersinn beschreibt. Als Belastung ist eine rein horizontal wirkende Kraft von 10 N angenommen, die im Punkt A (vgl. Figur 1), etwa in Höhe der Schneide eines Frontzahnes, angreift. Der Kurvenverlauf zeigt deutlich die hohen Spannungsspitzen, die in der Corticalis entstehen.

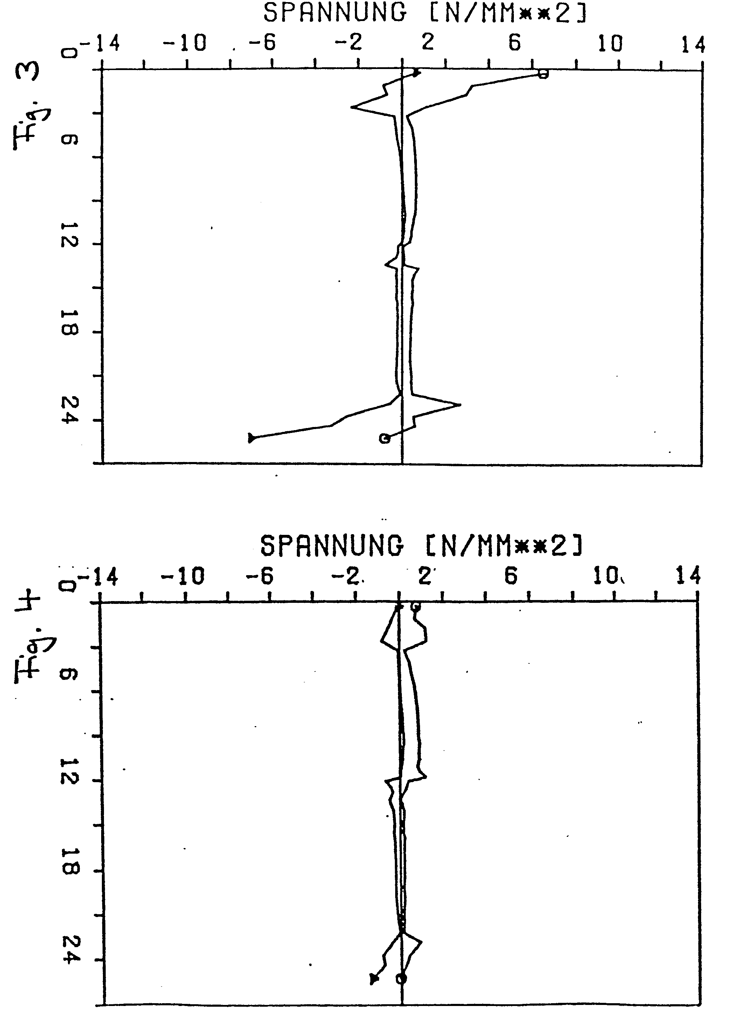

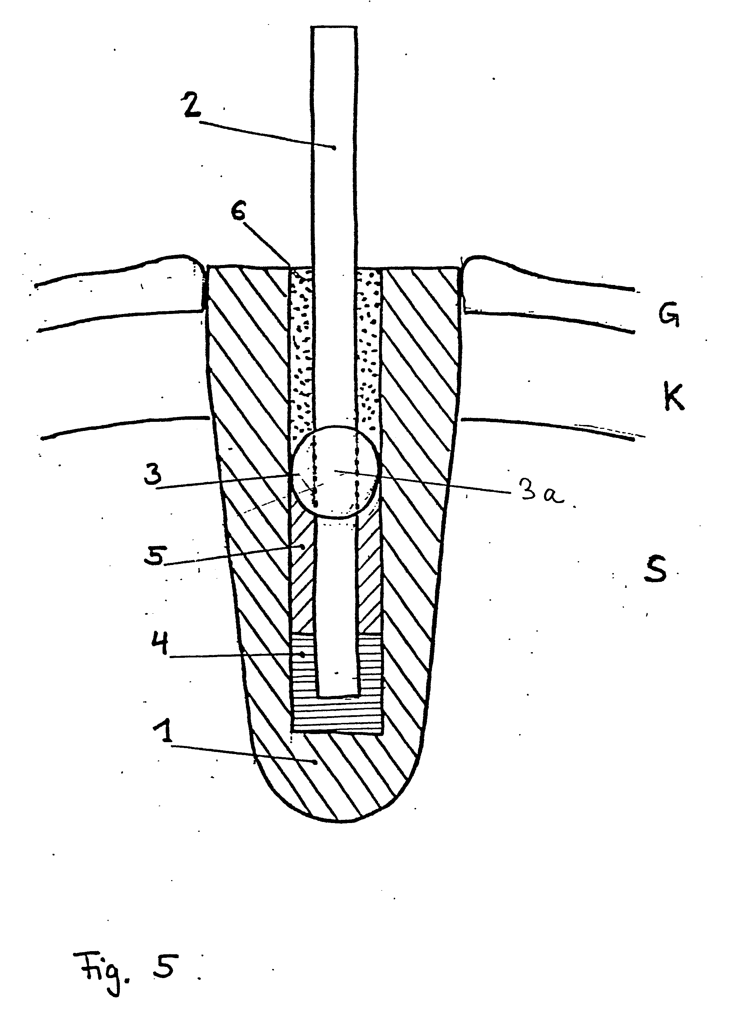

Figuren 3 und 4 zeigen die Spannungsverteilungen für den Fall, dass die Einleitung der Horizontalkraft nicht im Punkt A (vgl. Figur 1) erfolgt, sondern im Punkt B bzw. C, der etwa dem Rotationszentrum des Implantats entspricht.

Der Vergleich der Figuren 2 bis 4 lässt eindeutig erkennen, dass die am Durchtrittsrand des Implantats in den Knochen auftretenden Spannungsspitzen abhängig vom Ort der Krafteinleitung, d.h. vom aufgebrachten Drehmoment, sind. Greift die Kraft zum Beispiel in der Nähe des Rotationszentrums an, so können die Spannungen am Austrittsrand aus dem Knochen nahezu zum Verschwinden gebracht werden (Figur 4).

Vorschläge, die unphysiologischen Druckspitzen im Knochenbett zu reduzieren oder zu vermeiden, sind bereits bekannt. Sie gehen zumeist von der – physikalisch angreifbaren – Vorstellung aus, durch «kraft- bzw. stossdämpfende» Zwischenstücke, die sich «elastisch» verhalten (d.h. vermutlich: eine geringe Steifigkeit aufweisen), die Druckspitzen abbauen zu können. Diese Zwischenelemente bestehen entweder aus einem Aufsatz auf dem Implantat, der in die Suprastruktur hineinreicht, wie in DE-A 2 704 390, DE-A 2 824 214, DE-A 2 413 883, DE-A 2 950 219 und DE-A 2 830 025 beschrieben, und/oder aus einem Stift, wie in DE-A 2 824 214 beschrieben, oder einer Zwischenschicht im Innern des im Knochen liegenden Implantatteils, wie in DE-A 2 413 883, US-A 3 934 347 und EP-A 0 015 599 beschrieben, die jeweils aus einem weichen Kunststoff gefertigt sind. Alle diese Konstruktionen haben nur bedingt eine kraftumverteilende und damit die Spannungsspitzen reduzierende Wirkung, insofern nämlich, als sie aufgrund einer etwas erhöhten Beweglichkeit der Suprastruktur vor allem in vertikaler Richtung bei geeigneter Auslegung eine begrenzte Verteilung der angreifenden Kraft auf die Nachbarzähne oder weitere Pfeiler der Suprakonstruktion erlaubt. Die Gefährlichkeit der horizontalen Kraftkomponenten wird dagegen nicht oder nur geringfügig vermindert, da der Kraftangriffspunkt gleich bleibt oder nur wenig verlagert wird. Bei den Verbindungselementen mit Zwischenschicht zwischen dem Verbindungselement und dem Implantatkörper, wie sie z.B. in EP-A 0 015 599 beschrieben sind, treten ganz erhebliche Spannungsspitzen an der Implantataustrittsstelle aus dem Knochen auf, und von den Kräften, die an der Suprastruktur senkrecht zur Implantathauptachse angreifen, gelangt nur ein geringer Anteil in das Implantatinnere. Durch die bekannten Vorrichtungen kann somit ein Knochenabbau nicht vermieden werden.

Der Oberbegriff des Patentanspruchs 1 geht aus vom Gegenstand der US-A 3 934 347 oder der EP-A 0 015 599, aus denen ein Verbindungselement der eingangs genannten Art bekannt ist.

Der Erfindung liegt die Aufgabe zugrunde, die Nachteile der bekannten Vorrichtungen zu beheben und ein Verbindungselement für ein Implantat zu schaffen, mit dem speziell die Spannungsspitzen an der Implantataustrittsstelle aus dem Knochen vermindert bzw. vermieden und damit die Gefahren eines Knochenabbaus an dieser Stelle reduziert bzw. beseitigt werden.

Die Lösung dieser Aufgabe erfolgt erfindungsgemäss dadurch, dass der Stift eine Verdickung aufweist, über die der Stift im Einbauzustand im Implantatkörper unterhalb der Implantataustrittsstelle aus dem Knochen derart mit dem Inneren des Implantatkörpers in Verbindung steht, dass die an der Suprastruktur senkrecht zur Implantathauptachse angreifenden Kräfte in das Innere des Implantatkörpers umgeleitet werden.

Das erfindungsgemässe Verbindungselement für Implantate verteilt den Kraftfluss vom Ort der Krafteinleitung, z.B. dem Zahnersatz oder der Suprastruktur, in das Implantatlager, beispielsweise den Kieferknochen, so, dass lokale Überbelastungen des Knochens vermieden werden und eine in etwa gleichmässige Verteilung der vom Implantat im Knochenlager erzeugten Spannungen erreicht werden.

Das erfindungsgemässe Verbindungselement ist insbesondere für Zahnwurzelimplantate geeignet, jedoch auch für Gelenkersatz-Implantate.

Eine bevorzugte Ausführungsform des Verbindungselements nach der Erfindung zeichnet sich dadurch aus, dass der Durchmesser der Verdickung etwas kleiner als der Durchmesser der Bohrung ist.

Bei einem erfindungsgemässen Verbindungselement, bei dem eine Umhüllung vorgesehen ist, welche die Hohlräume zwischen dem Stift und der Innenwand der Bohrung mindestens teilweise ausfüllt, kann die Ausbildung so sein, dass

a) entweder am unteren Ende des Stifts eine Umhüllung aus einem Material mit höherem Elastizitätsmodul, als ihn der übrige Teil der Umhüllung besitzt, vorgesehen ist, wobei die am unteren Ende des Stifts befindliche Umhüllung einen Innendurchmesser besitzt, der grösser oder gleich dem Durchmesser des Stifts ist;

b) oder unterhalb der Verdickung eine Umhüllung aus einem Material mit höherem Elastizitätsmodul als ihn die Umhüllung besitzt, vorgesehen ist, wobei die Umhüllung einen Innendurchmesser besitzt, der grösser oder gleich dem Durchmesser des Stifts ist;

c) oder nur im unteren Bereich des Stifts eine Umhüllung vorgesehen ist, deren Elastizitätsmodul kleiner oder gleich dem des Stifts ist, wobei die Umhüllung einen Innendurchmesser besitzt, der grösser oder gleich dem Durchmesser des Stiftes ist.

d) oder an Stelle der Umhüllung die Bohrung des Implantatkörpers im unteren Teil zur Aufnahme des Stifts einen Querschnitt besitzt, dessen Durchmesser grösser oder gleich dem Durchmesser des Stiftes ist, und der übrige Teil der Umhüllung einen Elastizitätsmodul besitzt, der kleiner als der des Stifts und des Implantatkörpers ist.

Ausserdem wird mit der Erfindung ein Implantatkörper zur Verfügung gestellt, der ein erfindungsgemässes Verbindungselement aufweist.

Dieser Implantatkörper kann bevorzugt so ausgebildet sein, dass das Verbindungselement in den Implantatkörper eingesetzt, eingeschraubt oder eingeklebt ist.

Weiter ist es bei diesem Implantatkörper zu bevorzugen, dass restliche Hohlräume ganz oder teilweise mit einem plastisch verformbaren Material ausgefüllt sind, das sich unter Einsatzbedingungen oder durch Eigenschaftsänderungen nach dem Einbringen überwiegend elastisch verhält.

Das Verbindungselement nach der Erfindung kann insbesondere so ausgebildet sein, dass die Verdickung in vertikaler Richtung eine Durchbohrung aufweist, deren Durchmesser dem Durchmesser des Stifts entspricht. Hierbei kann der Stift ganz oder teilweise mit einem Aussengewinde und die Durchbohrung der Verdickung mit einem entsprechenden Innengewinde versehen sein. Insbesondere können dabei eine oder zwei Muttern, deren Innengewinde dem Gewinde des Stifts entspricht, am oberen oder/und unteren Teil der Verdickung vorgesehen sein.

Weiterhin kann das Verbindungselement zusätzlich mit einer oder mehreren Phasen oder Nuten versehen sein oder ein Aussengewinde aufweisen, das dem Innengewinde des Implantats entspricht.

Andere Weiterbildungen der Erfindung sind in den abhängigen Ansprüchen angegeben.

Die Erfindung wird nachstehend unter Bezugnahme auf die schematischen Abbildungen der Figuren anhand von Ausführungsbeispielen näher erläutert; es zeigen:

Fig. 1 bis 4 ein Modell eines Implantats und die Ergebnisse von Modellrechnungen über die Spannungsverteilungen bei einem solchen Modell;

Fig. 5 einen Querschnitt durch eine Ausführungsform eines erfindungsgemässen Verbindungselements;

Fig. 6, 7 und 8 Anordnungen zur Aufnahme der Suprastruktur;

Fig. 9 einen Querschnitt durch eine bevorzugte Ausführungsform der Erfindung; und

Fig. 10 einen Querschnitt durch eine weitere bevorzugte Ausführungsform der Erfindung.

In Fig. 5 ist der Implantatkörper mit 1 bezeichnet. Das Verbindungselement für den mit einer Bohrung versehenen Implantatkörper 1 und eine (nicht dargestellte) Suprastruktur, umfasst einen Stift 2 aus einem Werkstoff mit hohem E-Modul und eine Verdickung 3, die aus dem gleichen oder einem anderen Material wie der Stift 2 besteht.

Das Verbindungselement kann eine Umhüllung 4, 5, 6 mit einer Form aufweisen, die der Form der Bohrung des Implantats entspricht, und den Stift 2 zusammen mit der Verdickung 3 umhüllt. Die Umhüllung besteht aus einem Material, das den gleichen oder einen niedrigeren E-Modul aufweist als das Material, aus dem der Stift 2 hergestellt ist.

Der Implantatkörper kann allgemein aus jedem biokompatiblen (gewebsverträglichen) oder bioaktiven (gewebsaktiven) Material, das die erforderliche mechanische Stabilität besitzt, bestehen, und er kann eine beliebige äussere Form aufweisen. Der Implantatkörper sitzt in einem angedeuteten Kiefer mit der äusseren harten Knochenschale K (Corticalis), der inneren weichen Knochensubstanz S (Spongiosa) und dem Zahnfleisch G (Gingiva). Er kann jedoch auch in jeder anderen Knochenstruktur sitzen.

Der Implantatkörper besitzt eine Innenbohrung zur Aufnahme des Verbindungselements. Er kann auch ein Innengewinde aufweisen.

Das Verbindungselement besteht aus einem Stift 2 aus einem Material mit hoher Biegefestigkeit, d.h. hohem Elastizitätsmodul. Beispiele für solche Materialien sind Metalle, wie beispielsweise chirurgische Stähle, Co-Cr-Basislegierungen, Edelmetall-Legierungen, oder Keramiken, Verbundwerkstoffe, wie beispielsweise kohlefaserverstärkte Kohlenstoffe oder Kunststoffe. Bevorzugte Materialien für den Stift sind die oben angegebenen Metalle.

Der Stift kann weiterhin aus einem homogenen Material hergestellt sein oder aus einem inhomogenen Verbundmaterial, so dass längs der Achse ein variierender Elastizitätsmodul auftritt. Bevorzugt ist der Stift 2 aus einem homogenen Material hergestellt.

Der Stift kann einen runden, ovalen, vieleckigen oder anderen beliebigen Querschnitt aufweisen. Er ist jedoch bevorzugt rund. Der Stift kann einen konstanten Durchmesser haben, oder er kann entlang seiner Längsachse kontinuierlich oder diskontinuierlich im Durchmesser variieren.

Gemäss einer bevorzugten Ausführungsform der Erfindung enthält der Stift ganz oder teilweise ein Aussengewinde. Bevorzugt weist der Stift auf seiner gesamten Länge ein Aussengewinde auf, so dass man, nachdem der Stift in das Implantat und dieses in den Knochen eingesetzt wurde, auf den aus dem Implantat herausragenden Stift den Zahnersatz bzw. den Gelenkersatz aufschrauben kann.

Der Stift 2 ist durch Kürzen an seinem oberen und unteren Ende der jeweiligen Länge des Implantats beliebig anpassbar. Der Stift 2 mit der Verdickung 3 wird der Länge des Implantats durch Kürzen seiner Enden so angepasst, dass die Verdickung 3 ca. 0 bis 40% der halben Länge der Innenbohrung des Implantatkörpers über der Mitte im enossalen Teil des Implantats liegt, in Sonderfällen auch unterhalb der Mitte. Die genaue Lage wird durch die Art der Suprakonstruktion und den Implantationsort bestimmt.

Der Stift 2 weist eine angepasste Verdickung 3 auf. Die Verdickung 3 kann aus einem Material hergestellt sein, welches den gleichen oder einen anderen Elastizitätsmodul wie der Stift hat. Die Verdickung kann beispielsweise aus den gleichen Metallen, Keramiken oder Verbundwerkstoffen wie der Stift oder aus Kunststoffen hergestellt sein. Bevorzugte Materialien für die Verdickung sind die für den Stift angegebenen Metalle.

Die Verdickung 3 kann kugel-, tonnen- oder zylinderförmig ausgebildet sein. Sie kann in ihrem Querschnitt so ausgebildet sein, dass sie direkt, wie in der Zeichnung dargestellt, an der Bohrung des Implantats anliegt. Zwischen der Wand der Bohrung und der Verdickung kann jedoch auch ein Spiel vorhanden sein, d.h. der Durchmesser der Verdickung kann etwas kleiner als der Durchmesser der aufnehmenden Bohrung sein. Das Spiel kann im Bereich von wenigen μm bis zu einigen Zehntel mm liegen. Bevorzugt liegt es im Bereich von 10 bis 50 μm. Gemäss einer bevorzugten Ausführungsform der Erfindung ist die Verdickung als Kugel ausgebildet. Die Kugel ist bevorzugt durchbohrt. Der Durchmesser der Durchbohrung der Kugel entspricht dem Durchmesser des Stifts.

Die Durchbohrung weist bevorzugt einen runden Querschnitt auf. Vorzugsweise besitzt die durchbohrte Verdickung ein Innengewinde, so dass die Verdickung auf dem Stift, der ein Aussengewinde hat, verschiebbar ist und, nachdem sie in die günstigste Stellung gebracht worden ist, auf dem Stift starr fixiert ist.

Ein besonders bevorzugtes erfindungsgemässes Verbindungselement ist ein Verbindungselement, bei dem der Stift über seine gesamte Länge ein Aussengewinde aufweist, eine Kugel als Verdickung vorgesehen ist, die in der Mitte durchbohrt ist und ein Innengewinde aufweist, so dass sie auf dem Stift durch eine oder zwei Muttern in beliebiger Lage fixiert werden kann.

Der Stift 2 und die Verdickung 3 sind mit einer Umhüllung 4, 5, 6 versehen. Die Umhüllung besteht aus einem geeigneten Material, das i.a. einen niedrigeren Elastizitätsmodul aufweist als der Stift und der Implantatkörper. Sie kann auch aus verschiedenen Materialien zusammengesetzt sein, wie es beispielsweise in der Zeichnung mit 4, 5 und 6 angedeutet ist, wobei dann einzelne Abschnitte der Umhüllung denselben Elastizitätsmodul wie Stift oder Implantatkörper besitzen können.

Beispiele für derartige Materialien sind Kunststoffe, wie beispielsweise Polyäthylen, Epoxidharze, Acrylharze, Siliconkautschuke o.ä., aber auch für einzelne Abschnitte die oben aufgeführten Metalle oder Keramiken.

Die Umhüllung ist bevorzugt so ausgestaltet, dass sie eine Führungskappe 4 aufweist, in die der Stift 2 geschoben wird und die dem zentrischen Einbringen des Stiftes 2 und dem Einhalten des Abstands des Stiftes 2 vom Implantatboden unter Beibehaltung der federnden Lagerung dient. Die Führungskappe 4 soll bevorzugt aus einem Material mit höherem Elastizitätsmodul als der übrige Teil der Umhüllung bestehen.

Gemäss einer Ausführung der Erfindung besteht die Umhüllung nur aus der Führungskappe 4. Gemäss einer anderen Ausführungsform ist die Führungskappe 4 nicht vorhanden, und stattdessen ist die Bohrung im Implantat so ausgebildet, dass die Form der Bohrung der Führungskappe 4 entspricht. In anderen Worten besitzt die Bohrung des Implantatkörpers im unteren Teil einen Querschnitt, der dem Querschnitt des Stiftes entspricht.

Die Umhüllung 4, 5, 6 kann eine beliebige Form aufweisen. Der Querschnitt kann kreisförmig, oval, rechteckig oder durch eine Kombination dieser Formen entstanden sein. Die Umhüllung besitzt bevorzugt die gleiche Form wie die Bohrung des Implantats, so dass sie in diese hineinpasst.

Die Umhüllung kann in das Implantat einfach eingepasst, eingeklebt oder mittels eines Innengewindes in die Bohrung eingeschraubt werden, wie es für einen Teil 9 der Umhüllung in Figur 10 angedeutet ist.

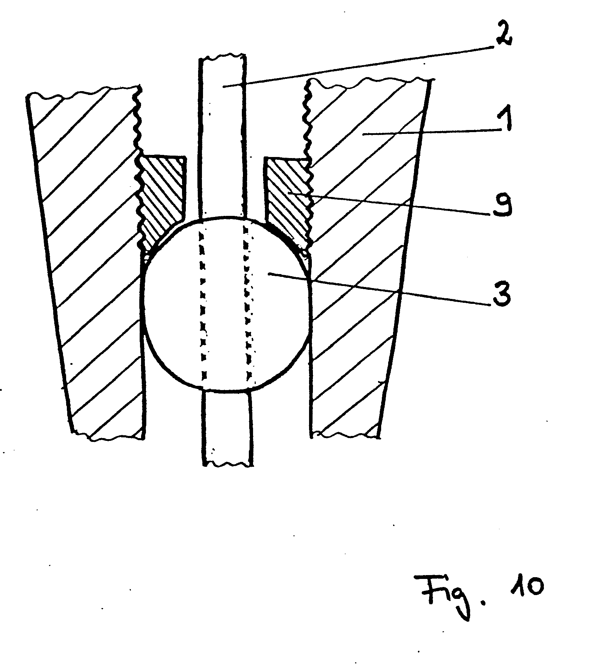

Die Umhüllung kann aus einem einheitlichen Material bestehen oder aus Materialien mit unterschiedlichem Elastizitätsmodul. Abschnittsweise kann auch die Umhüllung weggelassen werden, um unterschiedliche Verformungseigenschaften für unterschiedliche Belastungsrichtungen zu erhalten. Einzelne oder alle Abschnitte der Umhüllung können aus einem oder verschiedenen Materialien mit nichtlinearem Verformungsverhalten gefertigt sein, um die Beweglichkeit dem nichtlinearen Bewegungsverhalten natürlicher Zähne anzupassen. Zwischen dem Stift und der Umhüllung kann abschnittsweise, wie in Figur 9 dargestellt, Spiel herrschen, um ebenfalls die Beweglichkeit dem nichtlinearen Bewegungsverhalten der natürlichen Zähne anzunähern.

Die Umhüllung 4, 5, 6 kann mit dem Stift und der Verdickung fest oder verschieblich verbunden sein.

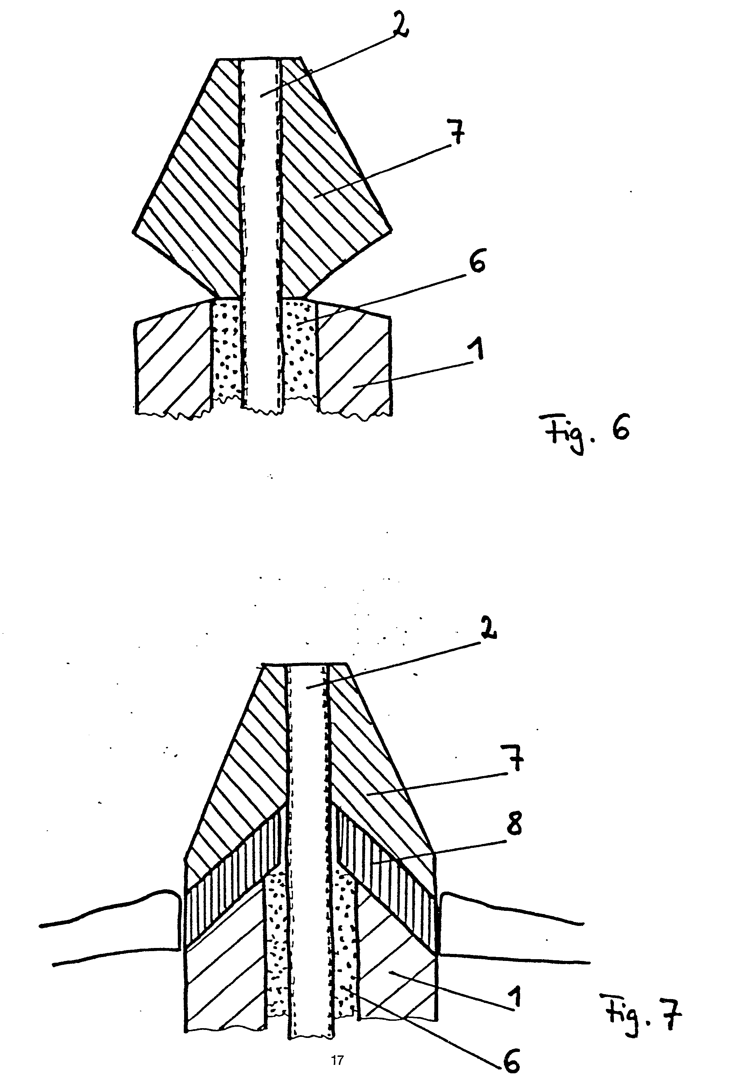

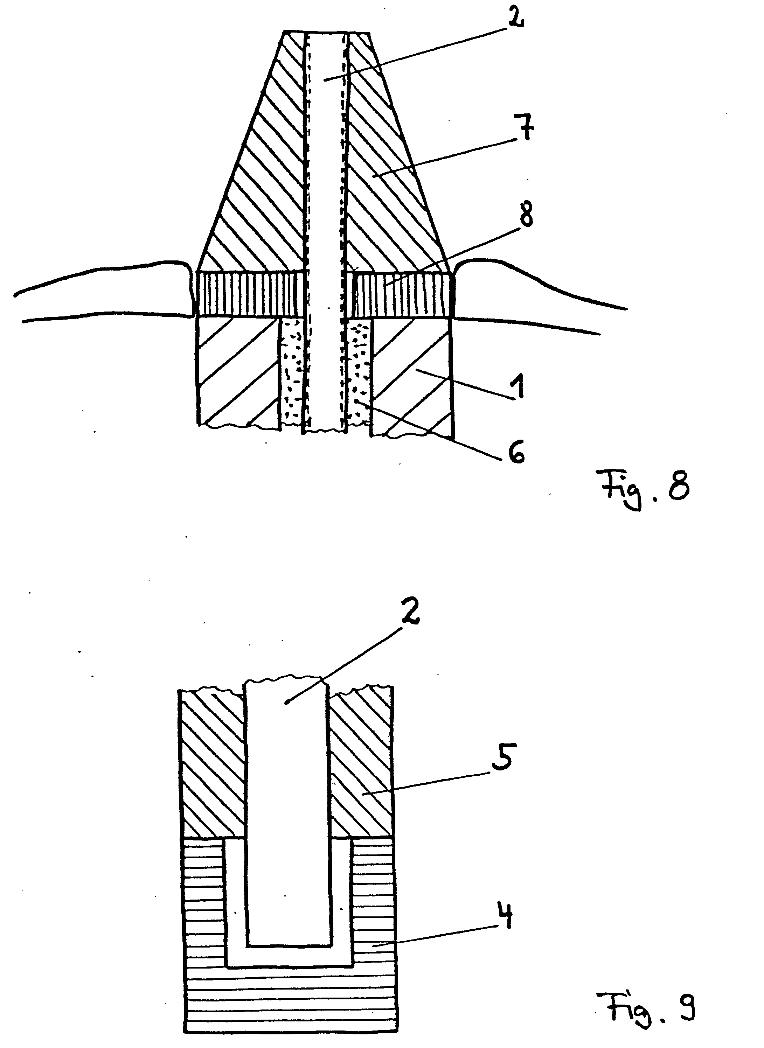

Der Stift wird zusammen mit der Umhüllung in das Implantat so eingesetzt, dass ein Teil des Stiftes herausragt. Auf den herausragenden Teil des Stiftes 2 wird ein Aufsatz 7 zur Anpassung der Suprastruktur befestigt. Dieser Aufsatz kann, vorzugsweise für abnehmbaren Zahnersatz, wie in Figur 6 dargestellt, auf der dem Implantat zugewandten Seite eine konische oder verrundete Einschnürung aufweisen, in die die Gingiva einwachsen kann. Für eine festsitzende Suprastruktur kann der Aufsatz 7 auf der dem Implantat zugewandten Seite eine ebene Fläche, wie in Figur 8 dargestellt ist, oder einen Innenkonus, wie in Figur 7 dargestellt ist, mit einem Winkel bis zu 45° besitzen, wobei in beiden Fällen eine entsprechend geformte weiche Dichtung 8 das Implantatinnere schützt. Die Anpassung an die Suprastruktur kann durch beliebige bekannte Massnahmen erfolgen, hier am Beispiel eines Konus gezeigt.

Als Suprastruktur können erfindungsgemäss jeder fest sitzende oder abnehmbare Zahnersatz oder Teile für den Gelenkersatz verwendet werden. Bevorzugt werden als Suprastruktur Kronen, Brücken oder Zahntotalprothesen verwendet.

Das erfindungsgemässe Verbindungselement führt in Verbindung mit einer Suprastruktur, die eine ideal starre Verbindung zu Nachbarzähnen oder -implantaten herstellt (zum Beispiel durch eine Brücke), zu einer Umlagerung einer an der Implantatkrone angreifenden Horizontalkraft. Dabei wird einmal durch Abstützen auf dem Nachbarpfeiler das aufgebrachte Drehmoment kompensiert, wobei es allerdings zu einer zusätzlichen Vertikalbelastung des Nachbarpfeilers kommt, die von den Dimensionen der Suprastruktur abhängt. Ausserdem verteilt sich die Horizontalkraft zu gleichen Teilen auf beide bzw. alle Pfeiler, wobei der für das Implantat verbleibende Anteil jetzt in Höhe der Verdickung angreift. Ist die Verdickung, vorzugsweise die Kugel, im Rotationszentrum des Implantats angebracht, so ergibt sich eine Spannungsverteilung wie in Figur 4 mit nahezu gleichmässig verteilten, niedrigen Spannungen im Knochenlager.

Bei der Anwendung des erfindungsgemässen Verbindungselements auf den Einzelzahnersatz ergeben sich ebenfalls günstigere Verhältnisse als beim starren Implantat. Wie FE-Rechnungen zeigen, wird aufgrund der Verformungseigenschaften des Verbindungselements auch hier eine Spannungsumverteilung bewirkt, die zu einer Reduzierung der Spannungsspitzen um mindestens die Hälfte gegenüber einem vergleichbaren starren Implantat führen, also etwa zu Verhältnissen, wie sie in Figur 3 gezeigt sind. Das ist zum Beispiel dann der Fall, wenn der unterhalb der Verdickung befindliche Teil der Umhüllung einen höheren Elastizitätsmodul als der obere Teil besitzt, so dass ein Moment grösstenteils in den unteren Teil des Implantats eingeleitet wird.

**Patentansprüche**

1. Verbindungselement für einen mit einer Bohrung versehenen Implantatkörper (1) mit einer Suprastruktur (7), wobei das Verbindungselement einen Stift (2) hoher Biegefestigkeit aufweist, dessen Durchmesser kleiner als derjenige der Bohrung ist und der in dem Hohlraum zwischen dem Stift (2) und der Wand des Implantatkörpers (1) mit letzterem in Verbindung steht, dadurch gekennzeichnet, dass der Stift (2) eine Verdickung (3) aufweist, über die der Stift im Einbauzustand im Implantatkörper (1) unterhalb der Implantataustrittstelle aus dem Knochen derart mit dem Inneren des Implantatkörpers (1) in Verbindung steht, dass die an der Suprastruktur (7) senkrecht zur Implantathauptachse angreifenden Kräfte in das Innere des Implantatkörpers (1) umgeleitet werden.

2. Verbindungselement nach Anspruch 1, dadurch gekennzeichnet, dass der Durchmesser der Verdickung (3) etwas kleiner als der Durchmesser der Bohrung ist.

3. Verbindungselement nach den Ansprüchen 1 und 2, wobei eine Umhüllung (4, 5, 6) vorgesehen ist, welche die Hohlräume zwischen dem Stift (2) und der Innenwand der Bohrung mindestens teilweise ausfüllt, dadurch gekennzeichnet, dass

(a) entweder am unteren Ende des Stifts (2) eine Umhüllung (4) aus einem Material mit höherem Elastizitätsmodul als ihn der übrige Teil (5, 6) der Umhüllung (4, 5, 6) besitzt, vorgesehen ist, wobei die am unteren Ende des Stifts (2) befindliche Umhüllung (4) einen Innendurchmesser besitzt, der grösser oder gleich dem Durchmesser des Stifts (2) ist;

(b) oder unterhalb der Verdickung (3) eine Umhüllung (4) aus einem Material mit höherem Elastizitätsmodul als ihn die Umhüllung (6) besitzt, vorgesehen ist, wobei die Umhüllung (4) einen Innendurchmesser besitzt, der grösser oder gleich dem Durchmesser des Stifts (2) ist;

(c) oder nur im unteren Bereich des Stifts (2) eine Umhüllung (4 bzw. 4, 5) vorgesehen ist, deren Elastizitätsmodul kleiner oder gleich dem des Stifts (2) ist, wobei die Umhüllung (4 bzw. 4, 5) einen Innendurchmesser besitzt, der grösser oder gleich dem Durchmesser des Stifts (2) ist;

(d) oder an Stelle der Umhüllung (4) die Bohrung des Implantatkörpers im unteren Teil zur Aufnahme des Stifts (2) einen Querschnitt besitzt, dessen Durchmesser grösser oder gleich dem Durchmesser des Stifts ist, und der übrige Teil (5, 6) der Umhüllung einen Elastizitätsmodul besitzt, der kleiner als der des Stifts (2) und des Implantatkörpers (1) ist.

4. Verbindungselement nach Anspruch 2 oder 3, dadurch gekennzeichnet, dass die Verdickung (3) kugel-, tonnen- oder zylinderförmig ausgebildet ist.

5. Verbindungselement nach Anspruch 2, 3 oder 4, dadurch gekennzeichnet, dass die Verdickung (3) in vertikaler Richtung eine Durchbohrung (3a) aufweist, deren Durchmesser dem Durchmesser des Stiftes (2) entspricht.

6. Verbindungselement nach Anspruch 5, dadurch gekennzeichnet, dass der Stift (2) ganz oder teilweise mit einem Aussengewinde und die Durchbohrung der Verdickung (3) mit einem entsprechenden Innengewinde versehen ist.

7. Verbindungselement nach Anspruch 6, dadurch gekennzeichnet, dass eine oder zwei Muttern, deren Innengewinde dem Gewinde des Stifts (2) entspricht, am oberen oder/und unteren Teil der Verdickung (3) vorgesehen sind.

8. Verbindungselement nach einem der vorhergehenden Ansprüche, dadurch gekennzeichnet, dass der Stift (2) einen konstanten Durchmesser aufweist oder entlang seiner Längsachse kontinuierlich oder diskontinuierlich im Durchmesser variiert.

9. Verbindungselement nach einem der vorhergehenden Ansprüche, dadurch gekennzeichnet, dass der Stift (2) aus einem Material mit konstantem oder längs der Achse variierendem Elastizitätsmodul gefertigt ist.

10. Verbindungselement nach einem der Ansprüche 3 bis 9, dadurch gekennzeichnet, dass die Umhüllung (4, 5, 6) aus Teilen mit unterschiedlichen Elastizitätsmodul zusammengesetzt ist oder aus einem oder verschiedenen Materialien mit nichtlinearem Verformungsverhalten besteht.

11. Verbindungselement nach mindestens einem der Ansprüche 1 bis 10, dadurch gekennzeichnet, dass sein äusserer Umfang einen kreisförmigen, ovalen, rechteckigen oder einen aus einer Kombination dieser Formen entstandenen Querschnitt aufweist, wobei der Querschnitt der Bohrung des Implantats entspricht.

12. Verbindungselement nach mindestens einem der Ansprüche 1 bis 11, dadurch gekennzeichnet, dass es zusätzlich mit einer oder mehreren Phasen oder Nuten versehen ist oder dass es ein Aussengewinde aufweist, das dem Innengewinde des Implantats entspricht.

13. Implantatkörper, dadurch gekennzeichnet, dass er das Verbindungselement nach einem der Ansprüche 1 bis 12 aufweist.

14. Implantatkörper nach Anspruch 13, dadurch gekennzeichnet, dass das Verbindungselement in den Implantatkörper (1) eingesetzt, eingeschraubt oder eingeklebt ist.

15. Implantatkörper nach Anspruch 13 oder 14, dadurch gekennzeichnet, dass restliche Hohlräume ganz oder teilweise mit einem plastisch verformbaren Material ausgefüllt sind, das sich unter Einsatzbedingungen oder durch Eigenschaftsänderungen nach dem Einbringen überwiegend elastisch verhält.

**Claims**

1. Connecting element for an implant body (1) with a bore and a superstructure (7), the connecting element having a pin (2) of high flexural strength, the diameter of which pin is smaller than that of the bore, said pin being in communication with the implant body (1) in the cavity between the pin (2) and the wall of said implant body (1), characterised in that the pin (2) has a thickening (3) by which the pin, when it is in position in the implant body (1), communicates with the interior of the implant body (1) below the point of exit of the implant from the bone so that the forces acting on the superstructure (7) in a direction perpendicular to the main axis of the implant are deflected to the interior of the implant body (1).

2. Conneting element according to claim 1, characterised in that the diameter of the thickening (3) is somewhat smaller than the diameter of the bore.

3. Connecting element according to claims 1 and 2 having a covering (4, 5, 6) at least partially filling the cavities between the pin (2) and the internal wall of the bore, characterised in that

(a) either a covering (4) made of a material with a higher modulus of elasticity than the remainder (5, 6) of the covering (4, 5, 6) is provided at the lower end of the pin (2), said covering (4) situated at the lower end of the pin (2) having an internal diameter greater than or equal to the diameter of the pin (2);

(b) or a covering (4) made of material having a higher modulus of elasticity than the covering (6) is provided below the thickening (3), the covering (4) having an internal diameter which is greater than or equal to the diameter of the pin (2);

(c) or a covering (4 or 4, 5) having a modulus of elasticity lower than or equal to that of the pin (2) is provided only in the lower region of the pin (2), said covering (4 or 4, 5) having an internal diameter which is greater than or equal to the diameter of the pin (2);

(d) or, instead of the covering (4), the bore of the implant body has, in its lower part, for receiving the pin (2), a cross-section whose diameter is greater than or equal to the diameter of the pin, and the remaining part (5, 6) of the covering has a modulus of elasticity which is lower than that of the pin (2) and of the implant body (1).

4. Connecting element according to claim 2 or 3, characterised in that the thickening (3) is spherical, barrel-shaped or cylindrical.

5. Connecting element according to claim 2, 3 or 4, characterised in that the thickening (3) is perforated in the vertical direction by a bore (3a) the diameter of which corresponds to the diameter of the pin (2).

6. Connecting element according to claim 5, characterised in that the pin (2) is covered partly or completely with an external thread and the continuous bore of the thickening (3) has a corresponding internal thread.

7. Connecting element according to claim 6, characterised in that one or two nuts whose internal thread corresponds to the thread of the pin (2) is or are provided in the upper and/or lower part of the thickening (3).

8. Connecting element according to one of the preceding claims, characterised in that the pin (2) has a constant diameter or varies continuously or stepwise in diameter along its longitudinal axis.

9. Connecting element according to one of the preceding claims, characterised in that the pin (2) is manufactured from a material having a modulus of elasticity which is constant or varies along the axis.

10. Connecting element according to one of the claims 3 to 9, characterised in that the covering (4, 5, 6) is composed of parts with different modul of elasticity or consists of one or different materials having a non-linear deformation characteristic.

11. Connecting element according to at least one of the claims 1 to 10, characterised in that its external surface has circular, oval or rectangular cross-section or a cross-section composed of a combination of these forms, the said cross-section corresponding to the bore of the implant.

12. Connecting element according to at least one of the claims 1 to 11, characterised in that it has, in addition, one or more phases or grooves or that it has an external thread which corresponds to the internal thread of the implant.

13. Implant body, characterised in that it has the connecting element according to one of the claims 1 to 12.

14. Implant body according to claim 13, characterised in that the connecting element is inserted into, screwed into or glued into the implant body (1).

15. Implant body according to claim 13 or 14, characterised in that the remaining cavities are partly or completely filled with a plastically deformable material which is predominantly elastic in behaviour under the conditions of use or due to changes in properties after it has been introduced.

**Revendications**

1. Elément de liaison pour un corps d'implantation ou implant (1) comportant un alésage, pourvu d'une superstructure (7), l'élément de liaison comportant une broche (2) de grande résistance à la flexion dont le diamètre est inférieur à celui de l'alésage et qui est, dans la cavité comprise entre la broche (2) et la paroi du corps d'implantation (1), en liaison avec ce dernier, caractérisé en ce que la broche (2) comporte un épaississement (3) par l'intermédiaire duquel la broche est, dans l'état d'insertion dans le corps d'implantation (1) au-dessous du point de sortie de l'implant de l'os, en liaison avec l'intérieur du corps d'implantation (1) de telle façon que les forces agissant sur la superstructure (7) orthogonalement à l'axe principal de l'implant sont détournées vers l'intérieur du corps d'implantation (1).

2. Elément de liaison selon la revendication 1, caractérisé en ce que le diamètre de l'épaississement (3) est un peu inférieur au diamètre de l'alésage.

3. Elément de liaison selon les revendications 1 et 2, une enveloppe (4, 5, 6) qui comble au moins partiellement les cavités comprises entre la broche (2) et la paroi intérieure de l'alésage étant prévue, caractérisé en ce que:

(a) ou bien il est prévu, à l'extrémité inférieure de la broche (2), une enveloppe (4) en une matière de module d'élasticité plus élevé que celui que possède le reste (5, 6) de l'enveloppe (4, 5, 6), l'enveloppe (4) se trouvant à l'extrémité inférieure de la broche (2) présentant un diamètre intérieur supérieur ou égal au diamètre de la broche (2);

(b) ou bien il est prévu, au-dessous de l'épaississement (3), une enveloppe (4) en une matière de module d'élasticité plus élevé que celui que possède l'enveloppe (6), l'enveloppe (4) présentant un diamètre intérieur supérieur ou égal au diamètre de la broche (2);

(c) ou bien il est prévu seulement dans la zone inférieure de la broche (2) une enveloppe (4, respectivement 4, 5) dont le module d'élasticité est inférieur ou égal à celui de la broche (2), l'enveloppe (4 ou 4, 5) présentant un diamètre intérieur supérieur ou égal au diamètre de la broche (2);

(d) ou bien, à la place de l'enveloppe (4), l'alésage du corps d'implantation présente, dans la partie inférieure destinée à recevoir la broche (2), une section droite dont le diamètre est supérieur ou égal au diamètre de la broche, et le reste (5, 6) de l'enveloppe présente un module d'élasticité inférieur à celui de la broche (2) et du corps d'implantation (1).

4. Elément de liaison selon la revendication 2 ou 3, caractérisé en ce que l'épaississement (3) a une forme sphérique, de barillet ou cylindrique.

5. Elément de liaison selon la revendication 2, 3 ou 4, caractérisé en ce que l'épaississement (3) comporte, en direction verticale, un perçage (3a) dont le diamètre correspond au diamètre de la broche (2).

6. Elément de liaison selon la revendication 5, caractérisé en ce que la broche (2) comporte, en totalité ou en partie, un filetage et le perçage de l'épaississement (3) un taraudage correspondant.

7. Elément de liaison selon la revendication 6, caractérisé en ce qu'un ou deux écrous dont le taraudage correspond au filetage de la broche (2) sont prévus à la partie supérieure ou/et inférieure de l'épaississement (3).

8. Elément de liaison selon l'une des revendications précédentes, caractérisé en ce que la broche (2) présente un diamètre constant ou bien un diamètre variant de façon continue ou discontinue suivant son axe longitudinal.

9. Elément de liaison selon l'une des revendications précédentes, caractérisé en ce que la broche (2) est constituée par une matière de module d'élasticité constant ou variant suivant l'axe.

10. Elément de liaison selon l'une des revendications 3 à 9, caractérisé en ce que l'enveloppe (4, 5, 6) se compose de parties de modules d'élasticité différents, ou bien est constituée par une ou plusieurs matières à propriétés de déformation non-linéaires.

11. Elément de liaison selon l'une au moins des revendications 1 à 10, caractérisé en ce que sa périphérie extérieure présente une section droite circulaire, ovale, rectangulaire, ou bien correspondant à une combinaison de ces formes, la section droite correspondant à l'alésage de l'implant.

12. Elément de liaison selon l'une au moins des revendications 1 à 11, caractérisé en ce qu'il comporte, en plus, une ou plusieurs facettes ou rainures, ou en ce qu'il comporte un filetage qui correspond au taraudage de l'implant.

13. Corps d'implantation ou implant, caractérisé en ce qu'il comporte l'élément de liaison selon l'une des revendications 1 à 12.

14. Corps d'implantation selon la revendication 13, caractérisé en ce que l'élément de liaison est inséré, vissé ou collé dans le corps d'implantation (1).

15. Corps d'implantation selon la revendication 13 ou 14, caractérisé en ce que des cavités résiduelles sont comblées totalement ou partiellement par une matière déformable plastiquement, qui a des propriétés surtout élastiques dans les conditions d'utilisation ou en raison de variations de propriété après l'introduction.

Fig. 1

Legend:
- $Al_2O_3$ – Keramik
- Keramik – Umhüllung
- Corticalis
- Spongiosa

Fig. 2

SPANNUNG [N/MM**2]

Fig. 3

SPANNUNG [N/MM**2]

Fig. 4

Fig. 5

Fig. 6

Fig. 7

17

Fig. 8

Fig. 9

Fig. 10